# EUROPEAN PATENT APPLICATION

(11) **EP 1 935 452 A1**
(43) Date of publication of application: **25.06.2008**
(21) Application number: 06126455.2
(22) Date of filing: 19.12.2006
(51) Int. Cl.: A61N 5/06, G02F 1/15, A61B 18/20

(54) **Electrochromic device and photodynamic treatment device comprising such an electrochromic device**

(71) Applicant: Koninklijke Philips Electronics N.V., 5621 BA Eindhoven (NL)
(72) Inventor: The designation of the inventor has not yet been filed
(74) Representative: Schoenmaker, Maarten

(57) **Abstract**

Presently, many variations of light treatment are used in health care. Prime examples are the in-vivo or ex-vivo photodynamic treatment (PDT) of skin diseases, cancer/tumors, psoriasis, mood disorders, bladder infections, promoting wound closure, recovering spinal cord injuries, and countering muscle/bone atrophy. PDT is a treatment that uses a drug, called a photosensitizer or photosensitizing agent, and a particular type of light. The invention relates to an electrochromic device and to a photodynamic treatment device comprising such an electrochromic device.

## Description

### FIELD OF THE INVENTION

The invention relates to an electrochromic device. The invention also relates to a photodynamic treatment device, comprising such an electrochromic device.

### BACKGROUND OF THE INVENTION

Presently, many variations of light treatment are used in health care. Prime examples are the in-vivo or ex-vivo photodynamic treatment (PDT) of skin diseases, cancer/tumors, psoriasis, mood disorders, bladder infections, promoting wound closure, recovering spinal cord injuries, and countering muscle/bone atrophy. PDT is a treatment that uses a drug, called a photosensitizer or photosensitizing agent, and a particular type of light. When photosensitizers are exposed to a specific wavelength of light, they produce reactants, like oxygen radicals, that kills nearby cells. Each photosensitizer is activated by light of a specific wavelength. As this wavelength determines how far the light can travel into the body, specific photosensitizers and wavelengths of light are utilized to treat different areas of the body. Currently, most PDT devices employ a variety of different monochromatic light sources such as lasers or LED's and are able to cover a wavelength range from infrared (IR, 1100 nm) to ultra violet (UV, 300 nm). A major drawback of the known PDT device is that merely a single wavelength is available for photodynamic therapy. Since particular (biological) processes require certain (merely) specific wavelengths, the application of the known PDT devices is limited to the activation of photosensitizers falling within the wavelength range emitted by the PDT device. There is an increasing need to a PDT device which is adapted to both simultaneously and successively activate multiple photosensitizers requiring mutually different activation wavelengths or wavelength ranges.

It is an object of the present invention to provide an improved device with which multiple photosensitizers can be activated successively.

### SUMMARY OF THE INVENTION

This object can be achieved by providing an electrochromic device according to the preamble, comprising: at least one polychromatic lighting device, and at least one switchable electrochromic window for selectively regulating the transmission of at least one specific wavelength emitted by the lighting device in response to a voltage applied to the electrochromic window. Since the electrochromic window of the electrochromic device according to the invention experiences different opacities due to an electrochemical redox reaction within said electrochromic window caused by the application of a voltage, different light transmission characteristics (opacities) will be exhibited by the electrochromic window. By means of the switchable electrochromic device it is thus possible to selectively filter light emitted by the polychromatic lighting device, and hence to selectively transmit light with a particular wavelength or wavelength band depending on the voltage applied to the electrochromic window. By selectively switching and eventually regulating the electrochromic window, the electrochromic device according to the invention is adapted to effectively emit multiple wavelengths and/or multiple wavelength ranges (bands) - beside simultaneously - also successively. Hence, in case the device according to the invention is incorporated in a PDT, it is beneficially possible to successively activate multiple photosensitizers requiring mutually different activation wavelength(s). In this context it is noted that the expression 'light' must be interpreted rather broadly. This expression does not merely include electromagnetic radiation falling within the visible spectrum (typically having a wavelength range from 380 nm to 780 nm), but does also include non-visible electromagnetic radiation, such as infrared, ultraviolet, and may even include X-rays.

In an embodiment of the electrochromic device according to the invention, the lighting device comprises multiple monochromatic light sources. Examples of monochromatic light sources are a laser and a light emitting diode (LED). According to this embodiment it is preferred to apply multiple LED's, which are preferably stacked on top of each other. In a preferred embodiment, the lighting device comprises at least one polychromatic light source. In this manner, the lighting device, and hence the electrochromic device can be manufactured relatively compactly. Although a conventional (polychromatic) light bulb could be used in the device according to the invention, it is more preferable to apply one or multiple polychromatic organic LED's (OLED's) or one or multiple polychromatic plastic or polymer LED's (PLED's), although the production costs of the PLED are rather expensive. Polychromatic LED's are significantly more compact than conventional light bulbs. With respect to a conventional solid-state LED, an OLED has the potential to be able to be produced much more cheaply. Moreover, OLED's are lighter than LED's, and can be produced relatively easily by means of known deposition techniques. A typical OLED comprises an anode, a cathode, and at least two organic material layers disposed between the anode and cathode. The anode in many OLED's comprises a relatively high work function material, such as indium tin oxide (ITO), and the cathode typically comprises a relatively low work function material, such as calcium (Ca). One of the organic material layers in a typical OLED comprises a material having the ability to transport holes, and is thus typically referred to as a hole transport layer. Another organic material layer typically comprises a material having the ability to transport electrons, and is thus typically referred to as an electron transport layer. The electron transport layer may also function as the luminescent medium (or emissive layer). Alternatively, an additional emissive layer may be disposed between the hole transport layer and the electron transport layer. In either case, when the OLED is properly biased, the anode injects holes (positive charge carriers) into the hole transport layer, and the cathode injects electrons into the electron transport layer. The injected holes and electrons each migrate toward the oppositely charged electrode. When an electron and hole localize on the same molecule, a Frenkel excitation is formed, and (visible) light is emitted.

In a preferred embodiment the electrochromic device comprises multiple electrochromic windows. This embodiment may be advantageous in case a relatively large lighting area is desired or required, wherein the different electrochromic windows can be positioned adjacent to each other. In an alternative embodiment at least two electrochromic windows are adapted to exhibit different optical characteristics in response to a voltage applied to said windows. In accordance with this embodiment it could be advantageously to stack said mutually different electrochromic windows on top of each other. Since each electrochromic window can be switched on and off, and hence functions in fact as a switchable light filter, multiple switchable filters can be stacked on top of each other. This accumulative filtering may optimize and tune the transmission and hence the effective emission of a desired specific spectrum.

The electrochromic window preferably comprises a stack of multiple layers deposited onto a supporting surface. More particularly, the stack of multiple layers of the electrochromic window is deposited onto the lighting device, preferably formed by one or multiple LED's, in particular OLED's. The electrochromic window typically comprises a stack of an ion storage layer, an electrochromic layer, and a suitable solid-state electrolyte separating the ion storage layer and the electrochromic layer, wherein the choice of the material of the electrochromic layer is determining the wavelengths or wavelength ranges to be absorbed respectively to be transmitted by the electrochromic layer. The stack is suitable to be deposited onto a substrate. Commonly, a transparent current collector, such as e.g. indium tin oxide (ITO) is applied to the ion storage layer and the electrochromic layer respectively. The assembly of layers is normally enclosed by two glass layers. A power source is wired to the two ITO layers, and a voltage drives the ions from the ion storage layer, through the ion-conducting layer (electrolyte) and into the electrochromic layer. This makes the glass opaque. By tuning to the appropriate voltage, the ions are driven out of the electrochromic layers and into the ion storage layer. When the ions leave the electrochromic layer, the electrochromic window regains its transparency again.

The voltage required for powering the lighting device and the electrochromic window can be taken from an external power source, such as the electrical mains. However, in a preferred embodiment the electrochromic device comprises at least one electrochemical energy source for powering the at least one lighting device and the at least one electrochromic window. In a more preferred embodiment the electrochemical energy source makes integral part of the electrochromic device according to the invention. The electrochemical energy source preferably comprises at least one battery stack deposited onto a substrate, the battery stack comprising: a first battery electrode, a second battery electrode, and an intermediate solid-state electrolyte separating the first battery electrode and the second battery electrode. Such a thin-film battery stack can be fully integrated in a system-in-package (SiP) together with the lighting device and the electrochromic window. Preferably, the thin-film battery stack has a 3D orientation to improve the battery performance of the battery stack. Embodiments of 3D-oriented thin-film battery stack have already been described in the international patent application WO 2005/027245. Preferably, at least one electrode of the energy source according to the invention is adapted for storage of active species of at least one of following elements: hydrogen (H), lithium (Li), beryllium (Be), magnesium (Mg), aluminum (A1), copper (Cu), silver (Ag), sodium (Na) and potassium (K), or any other suitable element which is assigned to group 1 or group 2 of the periodic table. So, the electrochemical energy source of the energy system according to the invention may be based on various intercalation mechanisms and is therefore suitable to form different kinds of battery cells, e.g. Li-ion battery cells, NiMH battery cells, et cetera. In a preferred embodiment at least one electrode, more the battery anode, comprises at least one of the following materials: C, Sn, Ge, Pb, Zn, Bi, Sb, Li, and, preferably doped, Si. A combination of these materials may also be used to form the electrode(s). Preferably, n-type or p-type doped Si is used as electrode, or a doped Si-related compound, like SiGe or SiGeC. Also other suitable materials may be applied as anode, preferably any other suitable element which is assigned to one of groups 12-16 of the periodic table, provided that the material of the battery electrode is adapted for intercalation and storing of the abovementioned reactive species. The aforementioned materials are in particularly suitable to be applied in lithium ion based battery cells. In case a hydrogen based battery cell is applied, the positive electrode preferably comprises a hydride forming material, such as ABS-type materials, in particular LaNi5, and such as magnesium-based alloys, in particular MgxTil-x. The negative electrode for a lithium ion based cell preferably comprises at least one metal-oxide based material, e.g. LiCoO2, LiNiO2 LiMnO2 or a combination of these such as. e.g. Li(NiCoMn)02. In case of a hydrogen based energy source, the cathode preferably comprises Ni(OH)2 and/or NiM(OH)2, wherein M is formed by one or more elements selected from the group of e.g. Cd, Co, or Bi.

In a preferred embodiment of the electrochromic device according to the invention the electrochromic device comprises a control unit for controlling the voltage to be applied to the at least one electrochromic window. The control unit can also be powered by the integrated electrochemical energy source of the electrochromic device.

In order to secure a substantially homogeneous light output of the electrochromic device, preferably one or multiple optical foils are applied onto the at least one electrochromic window for diffusing light transmitted by said electrochromic window. These diffusing and/or reflecting foils are known in the prior art.

In a preferred embodiment the electrochromic device is partially surrounded by a packaging. The protective packaging is applied to prevent, or at least counteract, damaging of the electrochromic device. Since the packaging is commonly made of an opaque material, the electrochromic window is preferably left substantially uncovered by the packaging.

The invention also relates to a photodynamic treatment (PDT) device, comprising an electrochemical device according to the invention. To this end, the polychromatic lighting device is preferably chosen such that at least one photosensitizer can be activated by the wavelength(s) of the light emitted by said lighting device. In a preferred embodiment the PDT device is adapted for an in-vivo treatment of a human of animal body. More preferable, the PDT device is bioimplantable. In an alternative preferred embodiment the PDT is adapted for an ex-vivo treatment of a human or animal body. These (integrated or implantable) PDT devices can be advantageously used to for in-vivo or ex-vivo treatment of skin diseases, cancer/tumors, psoriasis, mood disorders, bladder infections, promoting wound closure, recovering spinal cord injuries, and countering muscle/bone atrophy.

### BRIEF DESCRIPTION OF THE DRAWINGS

The invention is illustrated by way of the following non-limitative examples, wherein:
Fig. 1 shows a schematic cross section of an electrochromic device according to the invention,
Fig. 2 shows a schematic cross section of another electrochromic device according to the invention,
Fig. 3a shows a detailed schematic cross section of a electrochromic window which could be used in the electrochromic device according to Figs. 1 and 2, and
Fig. 3b shows a detailed schematic cross section of another electrochromic window which could be used in the electrochromic device according to Figs. 1 and 2.

### DETAILED DESCRIPTION OF THE INVENTION

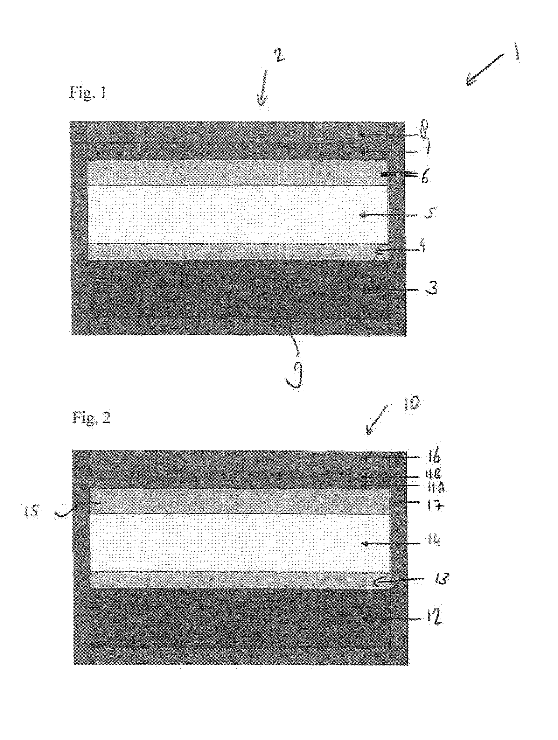

Fig. 1 shows a schematic cross section of an electrochromic device 1 according to the invention. The electrochromic device 1 shown in Fig. 1 is adapted to be used as a photodynamic treatment (PDT) device. The electrochromic device 1 comprises a laminate 2 of a thin-film solid-state battery 3 deposited onto a substrate (not explicitly shown), on top which battery 3 a first separation layer 4, a control unit 5, a second separation layer 6, a polychromatic OLED 7, and a electrochromic window 8 have been deposited successively. The integrated battery 3 is adapted for powering both the polychromatic OLED 7 and electrochromic window 8. The electrochromic window 8 is switchable by means of the control unit 5, and hence can be switched on and off. The electrochromic window 8 is switchable at least between a substantially transparent state and a state in which the window 8 is at least partially opaque. In this manner, the electrochromic window 8 will function in fact as a regulable light filter for selectively filtering light emitted by the polychromatic OLED 7, which makes it possible that the electrochromic device 1 will effectively emit light with a predefined wavelength or wavelength range, while other wavelengths (initially also emitted by the OLED 7) are absorbed by the electrochromic window 8. For a photodynamic treatment the electochromic device 1 can be controlled and switched such that different predefined wavelengths or different predefined wavelength ranges can be effectively emitted by the electrochromic device 1 either simultaneously or successively, which makes the electrochromic device 1 suitable for activating different photosensitizers either simultaneously or successively. The laminate 2 is surrounded by a protective packaging 9. The electrochromic device 1 can be adapted for ex-vivo use, but also for in-vivo use via bio implantation.

Fig. 2 shows a schematic cross section of another electrochromic device 10 according to the invention. The electrochromic device 10 shown in Fig. 2 is constructional substantially similar to the electrochromic device 1 shown in Fig. 1 with the difference that this electrochromic device 10 comprises multiple monochromatic LED's 11a, 11b stacked on top of each other, wherein the LED's 11a, 11b are adapted to emit light having a different color. The electrochromic device 10 further comprises a substrate on which a solid-state battery 12 is deposited, on top of which battery 12 a first separation layer 13, a control unit 14, a second separation layer 15, both monochromatic LED's 11 a, 11b, and a electrochromic window 16 have been deposited successively. A protective packaging 17 is applied to protect to the battery 12, the control unit 14, and the monochromatic LED's 11a, 11b. The monochromatic LED's 11 a, 11b together form a polymchromatic lighting device. The functioning of the electrochromic device 10 is similar to the electrochromic device 1 shown in Fig. 1.

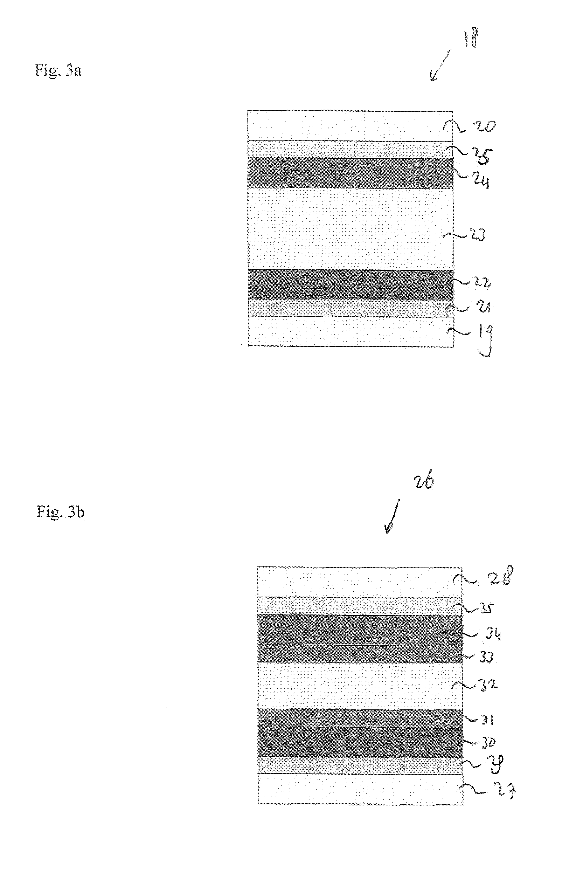

Fig. 3a shows a detailed schematic cross section of a electrochromic window 18 which could be used in the electrochromic device 1, 10 according to Figs. 1 and 2. The electrochromic window 18 shown in Fig. 3a is an electrochromic window 18 in which lithium-based active species are present. The electrochromic window 18 comprises two glass substrates 19, 20 between which a first current collector 21, an ion storage layer 22, an electrolyte 23, an electrochromic layer 24, and a second current collector 25 have been applied. In this example, the current collectors 21, 25 have been made of indium tin oxide (ITO). The ion storage layer 22 is formed by a CeO₂-TiO₂ layer, the electrolyte 23 is formed by LiPON, and the electrochromic layer 24 is formed by LiₓWO₃. A power source (not shown) is wired to the two current collectors 24, 25, and a voltage drives the lithium ions from the ion storage layer, through the ion-conducting layer 23 and into the electrochromic layer 24. This makes the electrochromic window 18 at least partially opaque. By tuning to the appropriate voltage, the ions are driven out of the electrochromic layer 24 and into the ion storage layer 22. When the ions leave the electrochromic layer 24, the electrochromic window 18 regains its transparency again.

Fig. 3b shows a detailed schematic cross section of another electrochromic window 26 which could be used in the electrochromic device l, 10 according to Figs. 1 and 2. The electrochromic window 26 shown in this Figure is an electrochromic window 26 in which hydrogen-based active species are present. The electrochromic window 26 comprises two glass substrates 27, 28 between which a first current collector 29, an ion storage layer 30, a first palladium layer 31, an electrolyte 32, a second palladium layer 33, an electrochromic layer 34, and a second current collector 35 have been applied. In this example, the current collectors 29, 35 are made of indium tin oxide (ITO). The ion storage layer 30 is formed by a Ni(OH)₂ layer, the electrolyte 32 is formed by ZrO_{y}Hₓ, and the electrochromic layer 34 is formed by Mg_{y}Gd_{(1-y)}Hₓ. The palladium layers 31, 33 are commonly ultra thin are adapted for catalyzing absorption and desorption of hydrogen at the ion storage layer 30 and the electrochromic layer 34.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The mere fact that certain measures are recited in mutually different dependent claims does not indicate that a combination of these measures cannot be used to advantage.

## Claims

1. Electrochromic device, comprising:
- at least one polychromatic lighting device, and
- at least one switchable electrochromic window for selectively regulating the transmission of at least one specific wavelength emitted by the lighting device in response to a voltage applied to the electrochromic window.

2. Electrochromic device according to claim 1, **characterized in that** the lighting device comprises multiple monochromatic light sources.

3. Electrochromic device according to claim 1 or 2, **characterized in that** the lighting device comprises at least one polychromatic light source.

4. Electrochromic device according to one of the foregoing claim 2 or 3, **characterized in that** the lighting device comprises at least one light emitting diode (LED).

5. Electrochromic device according to claim 4, **characterized in that** the LED is an organic LED (OLED).

6. Electrochromic device according to claim 4 or 5, **characterized in that** the lighting device comprises multiple LED's.

7. Electrochromic device according to one of the foregoing claims, **characterized in that** the electrochromic device comprises multiple electrochromic windows.

8. Electrochromic device according to claim 7, **characterized in that** at least two electrochromic windows are adapted to exhibit different optical characteristics in response to a voltage applied to said windows.

9. Electrochromic device according to one of the foregoing claims, **characterized in that** the at least one electrochromic window comprises a stack of multiple layers deposited onto a supporting surface.

10. Electrochromic device according to claim 9, **characterized in that** the stack of multiple layers is deposited onto the lighting device.

11. Electrochromic device according to one of the foregoing claims, **characterized in that** the electrochromic device comprises at least one electrochemical energy source for powering the at least one lighting device and the at least one electrochromic window.

12. Electrochromic device according to claim 11, **characterized in that** the electrochemical energy source comprises at least one battery stack deposited onto a substrate, the battery stack comprising: a first battery electrode, a second battery electrode, and an intermediate solid-state electrolyte separating the first battery electrode and the second battery electrode.

13. Electrochromic device according to one of the foregoing claims, **characterized in that** the electrochromic device comprises a control unit for controlling the voltage to be applied to the at least one electrochromic window.

14. Electrochromic device according to one of the foregoing claims, **characterized in that** the electrochromic device comprises at least one optical foil applied onto the at least one electrochromic window for diffusing light transmitted by said electrochromic window.

15. Electrochromic device according to one of the foregoing claims, **characterized in that** the electrochromic device is partially surrounded by a packaging.

16. Photodynamic treatment device, comprising an electrochromic device according to one of claims 1-15.

17. Photodynamic treatment device according to claim 16, **characterized in that** the photodynamic treatment device is adapted for an in-vivo treatment of a body.

18. Photodynamic treatment device according to claim 17, **characterized in that** the photodynamic treatment device is bioimplantable.

19. Photodynamic treatment device according to one of claims 16-18, **characterized in that** the photodynamic treatment device is adapted for an ex-vivo treatment of a body.
